# EUROPEAN PATENT SPECIFICATION

(11) **EP 2 193 421 B1**
(45) Date of publication and mention of the grant of the patent: **30.05.2012**
(21) Application number: 07808835.8
(22) Date of filing: 20.09.2007
(51) Int. Cl.: G06F 3/01, A61B 3/10, G01C 1/00, B60K 35/00, B60K 37/02, B60R 1/02, B60R 25/00, G02B 27/22, G06K 9/00, B60R 21/015, G01C 21/36, G02B 23/00, H04N 7/18, A61B 3/113, G01C 3/08, G02B 27/00

(54) **POSITION DETECTION ARRANGEMENT AND OPERATING METHOD FOR A POSITION DETECTION ARRANGEMENT**
POSITIONSDETEKTIONSANORDNUNG UND BETRIEBSVERFAHREN FÜR EINE POSITIONSDETEKTIONSANORDNUNG
SYSTEME DE DETECTION DE POSITION ET PROCEDE D'EXPLOITATION POUR UN SYSTEME DE DETECTION DE POSITION

(43) Date of publication of application: 09.06.2010
(73) Proprietor: Volvo Lastvagnar AB, 405 08 Göteborg (SE)
(72) Inventor: ANDERSSON, Lars, S-434 93 Vallda (SE)
(74) Representative: Fröhling, Werner Otto
(86) International application number: PCT/SE2007/000830
(87) International publication number: WO 2009/038504

(56) References cited:
- EP-A1- 1 800 964
- WO-A1-98/30414
- WO-A1-98/30414
- WO-A2-2006/108617
- US-A- 5 621 457
- US-A- 5 621 457
- US-A- 5 714 967
- US-A1- 2005 027 472
- US-A1- 2006 149 426

## Description

The invention relates to a position detection arrangement, an optical unit for a position detection arrangement and an operating method for a position detection arrangement according to the preambles of the independent claims.

Determination of a driver head position in a vehicle is known in the art. Information about the driver head position is necessary for e.g. systems comprising a head-up display for rendering optical information to the driver. It is known in the art to use video cameras for locating the position of the driver's eyes. According to this information, an appropriate area for displaying the information in the head-up display, e.g. on the windscreen, can be adjusted to the eyes' positions.

WO 98/30414 A1, considered as the closest prior art, discloses an apparatus and methods for automating the outside rearview mirror orientation of a vehicle. The apparatus comprises a light directing device which emits a visible light ray, orientation detectors, and electronic controller and a mirror positioning apparatus. The light directing device comprises a light source and a tube-like view restriction means which is pivotably mounted in a mount in the light directing device. In operation, the driver looks at the light directing device and orients the tube-like view restriction means so as to see the light ray emanating form the light source and then presses a switch to activate orientation sensors and distance sensors. The orientation sensors detect the orientation of the tube-like view restriction means in the mount, while the distance sensors are ultrasonic or infrared sensors.

In US 6,915,231 B1 an arrangement is disclosed where a rough estimation of a driver head position is extracted from inclination angles of the right and left external rear-view mirrors.

It is an object of the invention to provide an easy-to use and reliable position detection arrangement for a head position of a driver with an optical unit, as well as an appropriate optical unit for such an arrangement and an operating method for such an arrangement.

The objects are achieved by the features of the independent claims. The other claims and the description disclose advantageous embodiments of the invention.

A position detection arrangement for a head position of a driver is proposed, comprising an optical unit comprising at last one light source providing backlight directed to the driver and a mask device for blocking the light of the at least one light source when being arranged in an optical path between the at least one light source and the driver's head position, a manipulator device for accomplishing a relative movement between the at least one light source and a blocking member of the mask device, a computing unit coupled to the optical unit for capturing a distance between at least one of (a) two or more dedicated light-source positions, (b) two or more dedicated blocking member positions. The computing unit can also be used for calculating a position of the driver's head based on an angle between the positions and the driver's head position. Advantageously, the position of the at least one light source can be extracted from the optical unit as well as the position of the blocking member when blocking the light source. In this case, an optical path or optical axis can be defined from the light source through the blocking member to the driver's eye yielding a well defined direction from the light source to the driver's head position. Preferably, the light source can be a light emitting diode (LED) which features a high illuminating power. This is beneficial if the adjustment of the relative positions of the light source and the mask device have to be made in bright daylight.

The blocking member can be a mechanical shield or an electronic member, such as an LCD screen area which can provide an optically nontransparent or at least light-damping spot which blocks the light of the at least one light source when moved over the at least one light source.

The arrangement is compact and can be preferably integrated in already existing equipment of the vehicle, e.g. an instrument panel. Existing components in the vehicle can be used. Additional installation space can be minimized.

If the optical unit can be integrated in an instrument panel of a vehicle, the driver's head is in a normal position with respect to normal driving situations when calibrating the system by determining the position of the driver's head. This installation space can be easily accessed by the driver as it is in the driver's field of vision. It is possible, however, to install the arrangement in other installation spaces which can be accessed by the driver, preferably when the driver's head is in a desired position, i.e. in a position which the driver takes during driving.

The manipulator can be coupled to the mask device for moving the blocking member of the mask device. This means that the at least one light source is fixed and the blocking member is moved until it overlaps the light source. The manipulator can be anything suitable like a rocker switch or an electronic switch or a turning knob for moving the blocking member in the mask device to reach an overlapping position with the light source below the mask device.

Additionally or alternatively, the manipulator can be coupled to the light source for moving the light source. This means that the blocking member can be fixed and the light source is moved until the blocking member overlaps the light source. Moving the light source can be a mechanical movement of the light source or activating light sources in an appropriately dense array of light sources.

The mask device can favourably comprise an LCD-screen providing an optically nontransparent dot for blocking the light of the at least one light source. Shifting the dot to a desired position, i.e. to overlap with the light source in the field of view of the driver can be easily accomplished by electrical means known in the art. By providing an appropriate voltage to the pixels of the LCD screen a "black dot" can be provided which can block the light of the light source behind the LCD screen.

Advantageously, the at least one light source can be integrated in an array of light sources, particularly a two dimensional array of light sources. This allows for selecting an appropriate position of the at least one light source to be switched on for calibration of the head position as well as successively selecting two or more light sources to improve the accuracy of the position detection. Only one light source is active when positioning the blocking member accordingly (or vice versa). It is possible to make two or more subsequent measurements when one eye locates the light source and the blocking member and record an average value of the measurements. For a single measurement, one light source or one light source position, respectively, is chosen for one eye, i.e. one light source/position for the left eye and another light source/position for the right eye. This can be a right array for the right eye and a left array for the left eye if the optical unit exhibits two light source arrays. A high accuracy can be obtained if the at least one light source and/or a position of the at least one light source can be selected in the left area of the optical unit for the right eye of the driver and in the right area of the optical unit for the left eye of the driver. This can be done by choosing the left light source array for the right eye and the right light source array for the left eye of the driver if the optical unit comprises two light source arrays.

Additionally, a two-dimensional LED array enables space triangulation not only in a horizontal plane but also in a vertical plane for calculating the head position on all three dimensions.

According to another aspect of the invention, an optical unit is proposed which provides at least one light source providing a backlight directed to the driver and a mask device for blocking the light of the at least one light source when being arranged in an optical path between the at least one light source and the driver's head position. As elucidated above, the optical unit provides a compact and easy-to-use arrangement.

The at least one light source can be arranged in an array of light sources. This allows for flexibility in the geometrical arrangement and for flexibility in ranges of drivers' head positions.

Favourably, a selection unit, e.g. a switch, can be provided for selecting at least one light source in the array depending on an eye position of the driver and/or selecting an optically blocking member of the mask device.

The manipulator can be coupled to the mask device for moving the blocking member of the mask device. Additionally or alternatively, the manipulator can be coupled to the light source for moving the light source.

In a preferred embodiment, an area of the blocking member can smaller than a lighted area on the mask device which is illuminated by the at least one light source. Advantageously, this embodiment allows for an arrangement that the driver recognizes the light source, when blocked by the blocking member, as a lighted ring around the blocking member, yielding a kind of a "dioptre" vision of the light source. The human eye is sensible with respect to symmetries. By arranging the relative position of blocking member and active light source to yield a lighted ring around the blocking member, the blocking member can easily be adjusted with high accuracy without overburdening the driver with complicated fine tuning of the alignment.

The mask device can favourably comprise an LCD-screen providing an optically absorbing or nontransparent dot for blocking the light of the at least one light source.

A preferred distance of at least 0.5 cm, preferably of at least 1 cm, can be arranged between the at least one light source and the mask device. This allows for a reasonable large parallax when the adjustment of light source and mask device is performed individually for each eye of the driver, resulting in a reasonable accuracy in determining the head position.

According to a favourable embodiment, two light source arrays can be provided, with one array assigned to each one eye of the driver.

According to another aspect of the invention, a method for operating a position detection arrangement for a head position of a driver is proposed, wherein the following calibration steps are performed for each eye of the driver:
- receiving light from at least one light source of an optical unit providing backlight directed to the driver;
- blocking the light with a mask device arranged in an optical path between the at least one light source and the driver's head position;
- using spatially different light sources for each eye;
- capturing a distance between at least one of the two
   two or more dedicated light-source positions,
   two or more dedicated blocking member positions; and
- calculating a position of the driver's head based on an angle between the positions and the driver's head position. This allows for an easy-to-use determination of the driver's head position.

Advantageously, an optical axis based on a relative orientation of the at least one light source and a respective blocking member of the mask device can be determined when blocking the light of the respective at least one light source

The head position can be easily be decided by determining an intersection of at least two optical axes based on said relative positions and the distance between the dedicated positions. The head position, particularly the eye position of the driver, is located in the area of the intersection. This position can be forwarded to vehicular devices for an head-up display or for an airbag system which uses the head position for improving the driver's safety when igniting the airbag.

A high accuracy can be obtained if the at least one light source and/or a position of the at least one light source cam be selected in the left area of the optical unit for the right eye of the driver and in the right area of the optical unit for the left eye of the driver. This can be done by choosing the left light source array for the right eye and the right light source array for the left eye of the driver if the optical unit comprises two light source arrays. However, it is possible to use one single array in the same way if the optical unit comprises only one single array.

The at least one light source and/or a position of the at least one light source can be determined depending on which eye is used for calibrating the position detection arrangement.

The present invention together with the above-mentioned and other objects and advantages may best be understood from the following detailed description of the embodiments, but not restricted to the embodiments, wherein is shown schematically:
- Fig. 1: a sketch of a principal setup of a preferred arrangement for detecting a position of a driver's head;
- Fig. 2: a preferred integration of a position detection arrangement for a head position of a driver in an instrument panel of a vehicle;
- Fig. 3: a side cut through a preferred embodiment of an optical unit for a position detection arrangement for a head position of a driver;
- Fig. 4a, b, c: a top view on a preferred optical unit looking through a mask device placed over a light source array with a light source switched on (a) with an activated blocking member of a mask device moving towards a light spot of the light source, and with the blocking member in a blocking position blocking the light of the light source (c); and
- Fig. 5: a sketch for elucidating the computation of a head position.

In the drawings, equal or functionally similar elements are referred to by equal reference numerals. The drawings are merely schematic representations, not intended to portray specific parameters of the invention. Moreover, the drawings are intended to depict only typical embodiments of the invention and therefore should not be considered as limiting the scope of the invention.

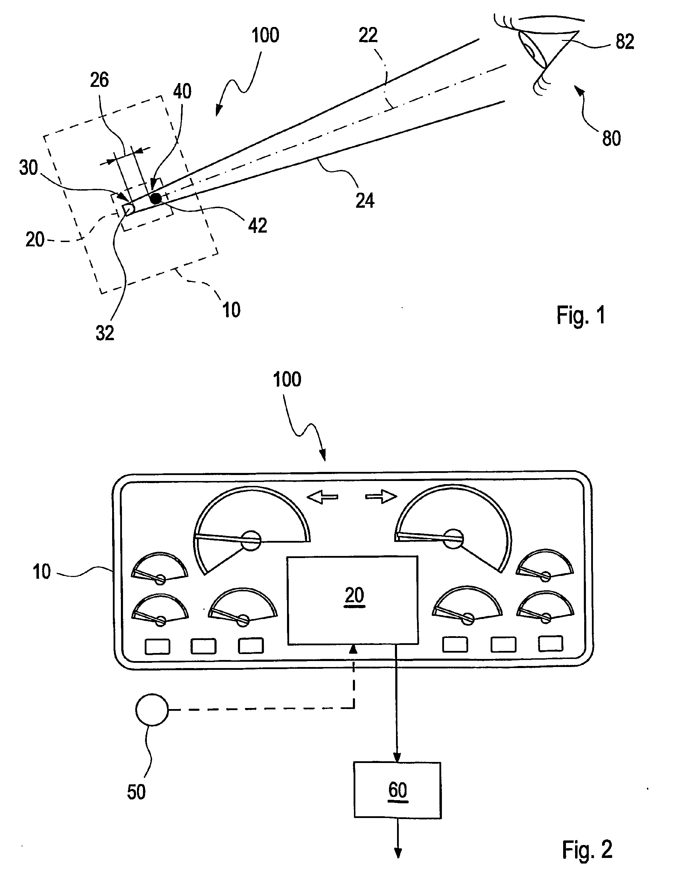

The schematic drawing in Fig. 1 elucidates the way of functioning of the invention by depicting a preferred embodiment of the arrangement 100. Fig. 2 depicts a preferred installation space of a preferred arrangement 100 for detecting a position of a driver's head in an instrument panel 10 of a vehicle where a manipulator 50 and a computing unit 60 are coupled to the optical unit 20.

The driver is looking onto an optical unit 20 which is preferably integrated into an instrument panel 10 of a vehicle, particularly of a light duty, medium or heavy duty commercial vehicle. The optical unit 20 is in the normal field of vision of the driver at the steering wheel.

Position signals generated in the optical unit 20 are transmitted to a computing unit 60 which receives and evaluates position information of the optical unit 20 and extracts information from this position information for determining the head position. The head position 80 is then transferred to other vehicular devices such as a head-up display which is readable by the driver when his head is in the detected position 80 or an airbag system, which can adjust to a driver's head position, which indirectly indicates the driver's size by the driver's head position.

For calibrating the arrangement 100, the driver looks with one eye 82 onto the optical unit 20. A light source 32, particularly an LED, emits light as a backlight directed to the driver's eyes. In front of the light source 32 in a distance 26 a mask device 40, particularly an LCD screen (LCD = liquid crystal display), is arranged with a blocking member 42. An LCD screen is transparent when no voltage is applied to the liquid crystal elements and becomes nontransparent or at least light absorbing when a certain voltage is applied. The light source 32 emits light in a light cone 24. The driver moves the blocking member 42 (i.e. "black dot") by using the manipulator 50 to overlap the light spot of the light source 32. It is also possible to move the light spot and keep the blocking member 42 fixed.

When the position of the light source 32 is known and the blocking position of the blocking member 42 relative to the light source 32, an optical axis 22 can be determined between the light source 32, the blocking member 42 and the driver's eye. The same is repeated by the driver with the other eye by activating another light source (not shown). This results in two spatially separated positions of one light source per eye and two spatially separate positions of one blocking member 42 per eye and two optical axes 22 from the two positions towards the driver's eye. The driver's eyes 82 represent the position 80 of the driver's head. The position 80 of the driver's head is calculated based on an angle between the spatially separate positions. Particularly, the position of the driver's eyes can be determined.

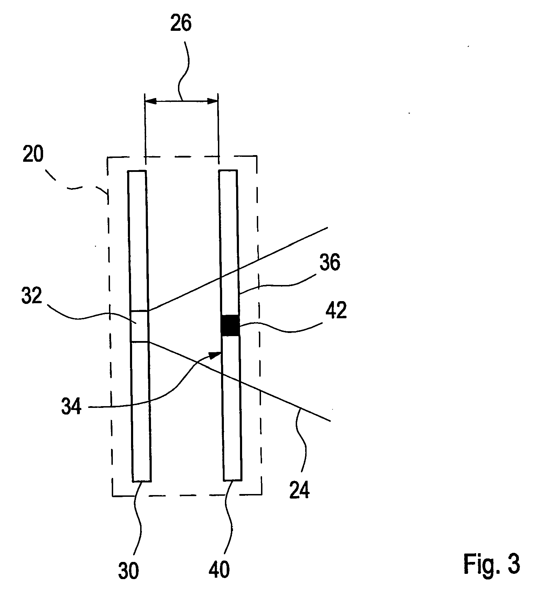

Fig. 3 depicts a side view of a cut through a preferred embodiment of an optical unit 20 for a position detection arrangement for a head position of a driver. The light source 32, e.g. an LED, can be preferably integrated in an array 30 with a multitude of horizontally and vertically spaced light sources. A mask device 40, e.g. an LCD screen, is arranged in a spatial distance 26 away from the array 30.

The light source 32 emits light typically in a light cone which appears as a light spot 34 on the mask device 40. A typical diameter of the LED light source is ca. 1 mm, whereas the light spot 34 could be 4-5 mm on the mask device 40. A reasonable value for the distance 26 provides a reliable parallax if the driver switches from one eye to the other looking onto the blocking member 42. With greater distance 26, the parallax becomes larger, i.e. if the driver looks with one eye at the blocking member 42, the position of the blocking member 42 apparently changes when looking with the other eye. The distance 26 is large enough to yield a reasonable parallax which allows for a reliable head position calculation even if the optical unit is operated by an inexperienced user. A favourable distance 26 can be at least 1 cm, favourably a value between 1 cm and 3 cm, particularly a value between 1 and 2 cm.

Preferably, the blocking member 42 is smaller in diameter than the light spot 34. With the blocking member 42 in its blocking position (for one eye), the light spot 34 appears as a ring 36 around the blocking member 42. This geometry makes use of the fact that the human eye is sensible to symmetries and the blocking member 42 can be arranged to overlap the light spot 34 with relatively high accuracy. A small shift would result in a clearly visible asymmetric appearance of the ring 36 and could be easily corrected.

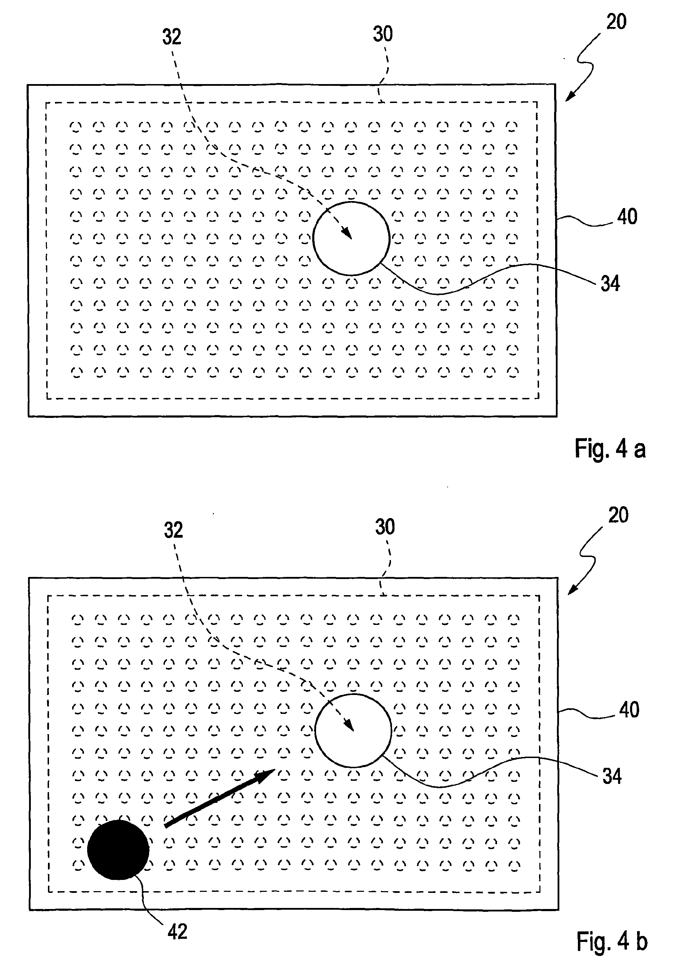

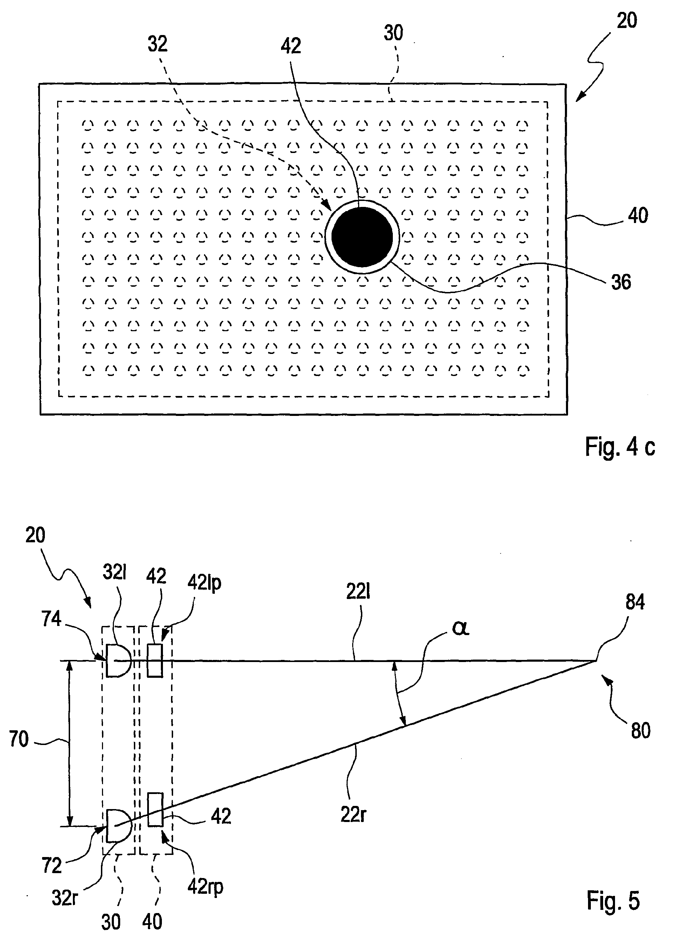

Fig. 4a through 4c show top views on an optical unit 20 with an array 30 of light sources 32 (only one is denoted with a reference numeral for clarity). The driver looks through the transparent mask device 40 onto the array 30. When the arrangement is to be calibrated, e.g. when starting the vehicle, one first light source 32 is switched on. The light source 32 casts a light spot 34 on the mask device 40 (Fig. 4a). A blocking member 42 is produced in the mask device 40 and moved towards the light spot 34 via manipulator 50 (Fig. 2) as indicated in Fig. 4b. While looking with one eye on the light spot 34, the driver can gauge the blocking member 42 in the centre of the light spot 34, thus producing a light ring 36 around the blocking member 42 (Fig. 4c). When the driver switches to the other eye, another light source positioned spatially apart from the first light source 32 is switched on (not shown) and the blocking member 42 is moved to overlap the new light spot as described above.

Depending on the size of the driver, his preferred sitting position behind the steering wheel and other like circumstances, the driver can choose an appropriately positioned light source 32 in the array 30 for performing the calibration. Further, instead of a single array 30, one array per eye can be provided in the optical unit 20 (Fig. 1), for instance a left array for the left eye and a right array for the right eye or vice versa. Favourably, the angles for calculating the head position are larger when the left array is used for the right eye and the right array for the left eye and thus, the calculation is more accurate.

Fig. 5 depicts a sketch for elucidating the computation of a driver's head position 84 with an optical unit 20 comprising a light source array 30 and a mask device 40. With one eye, e.g. the right eye, the driver locates a light source 32r and moves a blocking member 42 of a mask device 40 arranged in front of a light source array 30 and between the array 30 and the driver over this light source 32r to block the light of the light source 32r shining towards the driver. The position 72 of the light source 32r and the position 42rp of the blocking member 42 in the blocking position 42rp are recorded, e.g. in the computing unit 60 (Fig. 2).

Then the driver locates a spatially separated light source 321 with the left eye and blocks its light with the blocking member 42, e.g. moves the blocking member 42 from the blocking position 42rp to a position 421p where the light of the light source 321 shining towards the driver is blocked. The positions 421p of the blocking member and the position 74 of the light source 421 are recorded, too. As the positions 72, 74 of the light sources 32r, 321 are known, their distance 70 is known. The light sources 32r, 321 and the blocking positions 42rp, 421p define a right eye optical axis 22r and left eye optical axis 22r, which are inclined towards and intersect in the region of the driver's head. Because the head of the driver is relatively far away from the optical unit 20, the position of the two eyes can be merged in a single position with sufficient accuracy, yielding an angle α at the intersection 84. With the light source distance and the angle α the position of the driver's head compared with the optical unit 20 and the steering wheel can be determined.

## Claims

1. A position detection arrangement for a head position (80) of a driver, comprising an optical unit (20) comprising at least one light source (32, 32r, 32l) and a mask device (40) being arrangeable in an optical path (22, 22r, 22l) between the at least one light source (32, 32r, 32l) and the driver's head position (80),
**characterized in that**
- the optical unit (20) comprising the at least one light source (32, 32r, 32l) provides backlight directed to the driver and comprises the mask device (40), wherein the mask device (40) blocks the light of the at least one light source (32, 32r, 32l) when being arranged in an optical path (22, 22r, 22l) between the at least one light source (32, 32r, 32l) and the driver's head position (80);
- a manipulator device (50) is provided for accomplishing a relative movement between the at least one light source (32, 32r, 321) and a blocking member (42) of the mask device (40);
- a computing unit (60) is coupled to the optical unit (20) for capturing a distance (70) between at least one of
(a) two or more dedicated light-source positions (72, 74),
(b) two or more dedicated blocking member positions (42rp, 42lp);
and/or for calculating a position (80) of the driver's head based on an angle (α) between two or more dedicated light-source positions (72, 74) and the driver's head position (80) and/or between two or more dedicated blocking member positions (42rp, 421p) and the driver's head position (80).

2. The arrangement according to claim 1, **characterized in that** the optical unit (20) is integrated in an instrument panel (10) of a vehicle.

3. The arrangement according to claim 1 or 2, **characterized in that** the manipulator (50) is coupled to the mask device (40) for moving the blocking member (42) of the mask device (40).

4. The arrangement according to one of the preceding claims, **characterized in that** the manipulator (50) is coupled to the light source (32, 32r, 32l) for moving the light source (32, 32r, 32l).

5. An optical unit for a position detection arrangement (100) according to one of the preceding claims, **characterized by**
- at least one light source (32, 32r, 32l) providing a backlight directed to the driver,
- a mask device (40) for blocking the light of the at least one light source (32, 32r, 32l) when being arranged in an optical path (22, 22r, 22l) between the at least one light source (32, 32r, 321) and the driver's head position (80).

6. The optical unit according to claim 5, **characterized in that** the at least one light source (32, 32r, 321) is arranged in an array (30) of light sources (32).

7. The optical unit according to claim 5 or 6, **characterized by** a selection unit for selecting at least one light source (32, 32r, 32l) in the array (30) depending on an eye position (82) of the driver and/or selecting an optically blocking member (42, 42r, 42l) of the mask device (40).

8. The optical unit according to one of the claims 5 to 7, **characterized in that** the manipulator (50) is coupled to the mask device (40) for moving the blocking member (42) of the mask device (40), additionally or alternatively, the manipulator (50) is coupled to the light source (32, 32r, 321) for moving the light source (32, 32r, 321).

9. The optical unit according to one of the claims 5 to 8, **characterized in that** an area of the blocking member (42) is smaller than an lighted area (34) on the mask device (40) of the at least one light source (32, 32r, 321).

10. The optical unit according to one of the claims 5 to 9, **characterized in that** the mask device (40) comprises an LCD-screen providing an optically absorbing or nontransparent dot for blocking the light of the at least one light source (32, 32r, 321).

11. The optical unit according to one of the claims 5 to 10, **characterized in that** a distance (26) of at least 0.5 cm, preferably of at least 1 cm, is arranged between the at least one light source (32, 32r, 321) and the mask device (40).

12. A method for operating a position detection arrangement (100) for a head position (80) of a driver, comprising an optical unit (20) comprising at least one light source (32, 32r, 32l) and a mask device (40) being arrangeable in an optical patch (22, 22r 22l) between the at least one light source (32, 32r, 32l) and the driver's head position (80), **characterized by** for each eye of the driver
- receiving light from the at least one light source (32r, 321) of the optical unit (20) providing backlight directed to the driver;
- blocking the light with the mask device (40) arranged in an optical path (22l, 22r) between the at least one light source (32r, 32l) and the driver's head position (80);
- using spatially different light sources (32r, 32l) for each eye;
- capturing a distance (70) between at least one of
(a) two or more dedicated light-source positions (72, 74),
(b) two or more dedicated blocking member positions (42rp, 42lp); and
- calculating a position (80) of the driver's head based on an angle (α) between the positions (72, 74; 42rp, 42lp) and the driver's head position (80).

13. The method of claim 12, **characterized by** determining an optical axis (22r, 22l) based on a relative orientation of the at least one light source (32r, 32l) and a respective blocking member (42r, 421) of the mask device (40) when blocking the light of the respective at least one light source (32r, 32l).

14. The method of claim 13, **characterized by** determining an intersection (84) of at least two optical axes (22r, 22l) based on said relative positions and the distance (70) between the dedicated positions.

15. The method of one of the claims 12 to 14, **characterized by** selecting the at least one light source (32r, 321) and/or a position of the at least one light source (32r, 321) in the left area of the optical unit (20) for the right eye of the driver and in the right area of the optical unit (20) for the left eye of the driver.

## Patentansprüche

1. Positionserfassungseinrichtung für eine Kopfposition (80) eines Fahrers, mit einer optischen Einheit (20), die wenigstens eine Lichtquelle (32, 32r, 321) und eine Maskenvorrichtung (40) umfasst, die in einem optischen Weg (22, 22r, 221) zwischen der wenigstens einen Lichtquelle (32, 32r, 321) und der Kopfposition (80) des Fahrers anordbar ist,
**dadurch gekennzeichnet, dass**
- die die wenigstens eine Lichtquelle (32, 32r, 321) umfassende optische Einheit (20) ein auf den Fahrer gerichtetes Hintergrundlicht bereitstellt und die Maskenvorrichtung (40) umfasst, wobei die Maskenvorrichtung (40) das Licht der wenigstens einen Lichtquelle (32, 32r, 321) blockiert, wenn sie in einem optischen Weg (22, 22r, 221) zwischen der wenigstens einen Lichtquelle (32, 32r, 321) und der Kopfposition (80) des Fahrers angeordnet ist,
- eine Manipulatorvorrichtung (50) vorgesehen ist, um eine Relativbewegung zwischen der wenigstens einen Lichtquelle (32, 32r, 321) und einem Blockierelement (42) der Maskenvorrichtung (40) durchzuführen,
- eine Recheneinheit (60) mit der optischen Einheit (20) gekoppelt ist, um einen Abstand (70) zu erfassen zwischen wenigstens einem von
(a) zwei oder mehreren dedizierten Lichtquellenpositionen (72, 74),
(b) zwei oder mehreren dedizierten Blockelementpositionen (42rp, 42lp), und/oder um eine Position (80) des Kopfs des Fahrers auf der Basis eines Winkels (α) zwischen zwei oder mehreren dedizierten Lichtquellenpositionen (72, 74) und der Kopfposition (80) des Fahrers und/oder zwischen zwei oder mehreren dedizierten Blockelementpositionen (42rp, 42lp) und der Kopfposition (80) des Fahrers zu berechnen.

2. Einrichtung nach Anspruch 1, **dadurch gekennzeichnet, dass** die optische Einheit (20) in eine Instrumententafel (10) eines Fahrzeugs integriert ist.

3. Einrichtung nach Anspruch 1 oder 2, **dadurch gekennzeichnet, dass** die Manipulatorvorrichtung (50) mit der Maskenvorrichtung (40) zur Bewegung des Blockierelements (42) der Maskenvorrichtung (40) gekoppelt ist.

4. Einrichtung nach einem der vorhergehenden Ansprüche, **dadurch gekennzeichnet, dass** die Manipulatorvorrichtung (50) mit der Lichtquelle (32, 32r, 321) zur Bewegung der Lichtquelle (32, 32r, 321) gekoppelt ist.

5. Optische Einheit für eine Positionserfassungseinrichtung (100) nach einem der vorhergehenden Ansprüche, **gekennzeichnet durch**
- wenigstens eine Lichtquelle (32, 32r, 321), die ein auf den Fahrer gerichtetes Hintergrundlicht bereitstellt,
- eine Maskenvorrichtung (40) zur Blockierung des Lichts der wenigstens einen Lichtquelle (32, 32r, 321), wenn sie in einem optischen Weg (22, 22r, 221) zwischen der wenigstens einen Lichtquelle (32, 32r, 321) und der Kopfposition (80) des Fahrers angeordnet ist.

6. Optische Einheit nach Anspruch 5, **dadurch gekennzeichnet, dass** die wenigstens eine Lichtquelle (32, 32r, 321) in einer Anordnung (30) von Lichtquellen (32) angeordnet ist.

7. Optische Einheit nach Anspruch 5 oder 6, **gekennzeichnet durch** eine Wähleinheit zur Auswahl wenigstens einer Lichtquelle (32, 32r, 321) in der Anordnung (30) abhängig von einer Augenposition (82) des Fahrers und/oder zur Auswahl eines optisch blockierenden Elements (42, 42r, 421) der Maskenvorrichtung (40).

8. Optische Einheit nach einem der Ansprüche 5 bis 7, **dadurch gekennzeichnet, dass** die Manipulatorvorrichtung (50) mit der Maskenvorrichtung (40) gekoppelt ist, um das Blockierelement (42) der Maskenvorrichtung (40) zu bewegen, und zusätzlich oder alternativ die Manipulatorvorrichtung (50) mit der Lichtquelle (32, 32r, 321) zur Bewegung der Lichtquelle (32, 32r, 321) gekoppelt ist.

9. Optische Einheit nach einem der Ansprüche 5 bis 8, **dadurch gekennzeichnet, dass** ein Bereich des Blockierelements (42) kleiner ist als ein beleuchteter Bereich (34) an der Maskenvorrichtung (40) der wenigstens einen Lichtquelle (32, 32r, 321).

10. Optische Einheit nach einem der Ansprüche 5 bis 9, **dadurch gekennzeichnet, dass** die Maskenvorrichtung (40) einen LCD-Bildschirm umfasst, der einen optisch absorbierenden oder nicht transparenten Punkt zur Blockierung des Lichts der wenigstens einen Lichtquelle (32, 32r, 321) bereitstellt.

11. Optische Einheit nach einem der Ansprüche 5 bis 10, **dadurch gekennzeichnet, dass** ein Abstand (26) von wenigstens 0,5 cm, vorzugsweise von wenigstens 1 cm, zwischen der wenigstens einen Lichtquelle (32, 32r, 321) und der Maskenvorrichtung (40) vorgesehen ist.

12. Verfahren zum Betrieb einer Positionserfassungseinrichtung (100) für eine Kopfposition (80) eines Fahrers, die eine optische Einheit (20) umfasst, die wenigstens eine Lichtquelle (32, 32r, 321) und eine Maskenvorrichtung (40) umfasst, die in einem optischen Pfad (22, 22r, 221) zwischen der wenigstens einen Lichtquelle (32, 32r, 321) und der Kopfposition (80) des Fahrers anordbar ist, **dadurch gekennzeichnet, dass** für jedes Auge des Fahrers
- Licht von der wenigsten einen Lichtquelle (32r, 321) der optischen Einheit (20) empfangen wird, die ein auf den Fahrer gerichtetes Hintergrundlicht bereitstellt,
- das Licht mit der Maskenvorrichtung (40) blockiert wird, die in einem optischen Pfad (221, 22r) zwischen der wenigstens einen Lichtquelle (32r, 321) und der Kopfposition (80) des Fahrers angeordnet ist,
- räumlich unterschiedliche Lichtquellen (32r, 321) für jedes Auge verwendet werden,
- eine Distanz (70) erfasst wird zwischen wenigstens einem von
(a) zwei oder mehreren dedizierten Lichtquellenpositionen (72, 74),
(b) zwei oder mehreren dedizierten Blockelementpositionen (42rp, 421p), und
- eine Position (80) des Kopfs des Fahrers auf der Basis eines Winkels (α) zwischen den Positionen (72, 74; 42rp, 421p) und der Kopfposition (80) des Fahrers berechnet wird.

13. Verfahren nach Anspruch 12, **gekennzeichnet durch** die Bestimmung einer optischen Achse (22r, 221) auf der Basis einer relativen Ausrichtung der wenigstens einen Lichtquelle (32r, 321) und einem jeweiligen Blockierelement (42r, 421) der Maskenvorrichtung (40), wenn das Licht der jeweiligen wenigstens einen Lichtquelle (32r, 321) blockiert wird.

14. Verfahren nach Anspruch 13, **gekennzeichnet durch** die Bestimmung eines Schnittpunktes (84) wenigstens zweier optischer Achsen (22r, 221) auf der Basis der relativen Positionen und dem Abstand (70) zwischen den dedizierten Positionen.

15. Verfahren nach einem der Ansprüche 12 bis 14, **gekennzeichnet durch** die Auswahl der wenigstens einen Lichtquelle (32r, 321) und/oder einer Position der wenigstens einen Lichtquelle (32r, 321) in dem linken Bereich der optischen Einheit (20) für das rechte Auge des Fahrers und in dem rechten Bereich der optischen Einheit (20) für das linke Auge des Fahrers.

## Revendications

1. Agencement de détection d'une position pour une position de la tête d'un conducteur(80), comprenant une unité optique (20) comprenant au moins une source de lumière (32, 32r, 321) et un dispositif de masque (40) pouvant être agencé dans un trajet optique (22, 22r, 221) entre la au moins une source de lumière (32, 32r, 321) et la position de la tête d'un conducteur (80),
**caractérisé en ce que**
- l'unité optique (20) comprenant la au moins une source de lumière (32, 32r, 321) fournit un rétroéclairage dirigé vers le conducteur et comprend le dispositif de masque (40), le dispositif de masque (40) bloquant la lumière de la au moins une source de lumière (32, 32r, 321) lorsqu'il est agencé dans un trajet optique (22, 22r, 221) entre la au moins une source de lumière (32, 32r, 321) et la position de la tête d'un conducteur (80) ;
- un dispositif manipulateur (50) est agencé pour effectuer un mouvement relatif entre la au moins une source de lumière (32, 32r, 321) et un élément de blocage (42) du dispositif de masque (40) ;
- une unité de calcul (60) est couplée à l'unité optique (20) pour capturer une distance (70) entre au moins une position parmi
(a) deux ou plus de deux positions de source de lumière dédiée (72, 74),
(b) deux ou plus de deux positions d'élément de blocage dédié (42rp, 421p);
et/ou pour calculer une position (80) de la tête du conducteur sur la base d'un angle (α) entre deux ou plus de deux positions de source de lumière dédiée (72, 74) et la position de la tête du conducteur (80) et/ou entre deux ou plus de deux positions d'élément de blocage dédié (42rp, 421p)et la position de la tête du conducteur (80).

2. Agencement selon la revendication 1, **caractérisé en ce que** l'unité optique (20) est intégrée dans un panneau d'instruments (10) d'un véhicule.

3. Agencement selon les revendications 1 ou 2, **caractérisé en ce que** le manipulateur (50) est couplé au dispositif de masque (40) pour déplacer l'élément de blocage (42) du dispositif de masque (40).

4. Agencement selon l'une quelconque des revendications précédentes, **caractérisé en ce que** le manipulateur (50) est couplé à la source de lumière (32, 32r, 321) pour déplacer la source de lumière (32, 32r, 321).

5. Unité optique pour agencement de détection d'une position (100) selon l'une quelconque des revendications précédentes, **caractérisée par**
- au moins une source de lumière (32, 32r, 321) fournissant une lumière de rétroéclairage dirigée vers le conducteur ;
- un dispositif de masque (40) pour bloquer la lumière de la au moins une source de lumière (32, 32r, 321) lorsqu'il est agencé dans un trajet optique (22, 22r, 22l) entre la au moins une source de lumière (32, 32r, 32l) et la position de la tête du conducteur (80).

6. Unité optique selon la revendication 5, **caractérisée en ce que** la au moins une source de lumière (32, 32r, 321) est agencée dans un réseau (30) de sources de lumière (32).

7. Unité optique selon les revendications 5 ou 6, **caractérisée par** une unité de sélection pour sélectionner au moins une source de lumière (32, 32r, 321) du réseau (30) en fonction d'une position d'oeil (82) du conducteur et/ou sélectionner un élément optiquement bloquant (42, 42r, 421) du dispositif de masque (40).

8. Unité optique selon l'une quelconque des revendications 5 à 7, **caractérisée en ce que** le manipulateur (50) est couplé au dispositif de masque (40) pour déplacer l'élément bloquant (42) du dispositif de masque (40), en plus ou alternativement, le manipulateur (50) est couplé à la source de lumière (32, 32r, 321) pour déplacer la source de lumière (32, 32r, 321).

9. Unité optique selon l'une quelconque des revendications 5 à 8, **caractérisée en ce qu'**une surface de l'élément bloquant (42) est plus petite qu'une surface éclairée (34) sur le dispositif de masque (40) de la au moins une source de lumière (32, 32r, 321).

10. Unité optique selon l'une quelconque des revendications 5 à 9, **caractérisée en ce que** le dispositif de masque (40) comprend un écran LCD fournissant un point optiquement absorbant ou non transparent pour bloquer la lumière de la au moins une source de lumière (32, 32r, 321).

11. Unité optique selon l'une quelconque des revendications 5 à 10, **caractérisée en ce qu'**une distance (26) d'au moins 0,5 cm, de préférence d'au moins 1 cm, est agencée entre la au moins une source de lumière (32, 32r, 321) et le dispositif de masque (40).

12. Procédé d'actionnement d'un agencement de détection de position (100) pour une position de tête d'un conducteur (80), comprenant une unité optique (20) comprenant au moins une source de lumière (32, 32r, 321) et un dispositif de masque (40) pouvant être agencé dans un trajet optique (22, 22r, 221) entre la au moins une source de lumière (32, 32r, 321) et la position de la tête du conducteur (80), **caractérisé en ce que** pour chaque oeil du conducteur il consiste à:
- recevoir une lumière provenant de la au moins une source de lumière (32r, 321) de l'unité optique (20) fournissant un rétroéclairage dirigé vers le conducteur ;
- bloquer la lumière à l'aide du dispositif de masque (40) agencé dans un trajet optique (221, 22r,) entre la au moins une source de lumière (32r, 321) et la position de la tête du conducteur (80) ;
- utiliser des sources de lumière spatialement différentes (32r, 321) pour chaque oeil ;
- capturer une distance (70) entre au moins une position parmi
(a) deux ou plus de deux positions de source de lumière dédiée (72, 74),
(b) deux ou plus de deux positions d'élément bloquant dédié (42rp, 421p); et
- calculer une position (80) de la tête du conducteur sur la base d'un angle (α) entre les positions (72, 74 ; 42rp, 421p) et la position de la tête du conducteur (80).

13. Procédé selon la revendication 12, **caractérisé par** la détermination d'un axe optique (32r, 321) sur la base d'une orientation relative de la au moins une source de lumière (32r, 321) et d'un élément bloquant respectif (42r, 421) du dispositif de masque (40) lors d'un blocage de la lumière de la au moins une source de lumière (32r, 321).

14. Procédé selon la revendication 13, **caractérisé par** la détermination d'une intersection (84) d'au moins deux axes optiques (22r, 221) sur la base des positions relatives et de la distance (70) entre les positions dédiées.

15. Procédé selon l'une quelconque des revendications 12 à 14, **caractérisé par** une sélection de la au moins une source de lumière (32r, 321) et/ou d'urne position de la au moins une source de lumière (32r, 321) dans la surface gauche de l'unité optique (20) pour l'oeil droit du conducteur et dans la surface droite de l'unité optique (20) pour l'oeil gauche du conducteur.
